# EUROPEAN PATENT APPLICATION

(11) **EP 3 622 902 A1**
(43) Date of publication of application: **18.03.2020**
(21) Application number: 19197100.1
(22) Date of filing: 12.09.2019
(51) Int. Cl.: A61B 17/12

(54) **EMBOLIZATION COIL WITH END COIL INTEGRATED WITHIN MAIN COIL**

(30) Priority: 12.09.2018 US 201862730170 P
(71) Applicant: Cook Medical Technologies LLC, Bloomington, IN 47404 (US)
(72) Inventor: TEKULVE, Kurt J., Ellettsville, IN 47429 (US)
(74) Representative: Leach, Sean Adam

(57) **Abstract**

An improved occluding device (10) for occlusion of fluid flow through a lumen of a body vessel includes a main coil (12) having first primary coil windings (16) with a first coil diameter and an end coil (14) having second primary coil windings (22) with a second coil diameter. The main coil is formed into a secondary coil with various shapes. The main coil and the end coil are monolithically formed. A method of manufacturing the occluding device by a continuous process is also provided.

## Description

### FIELD OF THE INVENTION

The present invention relates to medical devices and, more particularly, to occluding devices and methods of making the devices.

### BACKGROUND OF THE INVENTION

Embolization coils (e.g. pushable fibered coils) have been used as a primary occluding device for treatment of various arteriovenous malformations (AVM) and varicoceles, as well as for many other arteriovenous abnormalities in the body. Occluding devices are also used to repair abnormal shunts between arteries and veins, prevent or reduce blood flow to tumors, stop hemorrhaging as a result of trauma, and stabilize aneurysms to prevent rupture. Embolization coils can be used to provide occlusion of fluid flow through a body vessel. Embolization coils are preferably used to occlude fluid flow through a body vessel due to a blood vessel malformation occurring in the brain, like aneurysms, or other part of the body. Occluding devices may vary for different purposes, e.g., to hold the device in place within a cavity or vessel and to pack the device within the vessel for enhanced occlusion.

Embolization coils may be configured in a variety of sizes and may be made of several different materials including stainless steel and platinum. Embolization coils can be built out of a main coil and an end coil. The end coil is a larger diameter coil and the main coil has a smaller diameter. The purpose of the end coil is to prevent a wire guide from getting wedged between the embolization coil and a catheter tubing when pushing the embolization coil through a catheter.

The current process for making an embolization coil with an end coil is to make the main coil and the end coil separately, and then attach the end coil to the proximal end of the main coil. The attachment can be a friction fit, a laser welded, adhesive, welded, soldered connection of the end coil to the main coil. However, there is a risk of detachment resulting in the end coil coming off the embolization coil and becoming a loose part. The separate processing steps of making the embolization coil may also involve an undesirable amount of time and cost.

Accordingly, improvements can be made in embolization coils and the process of making such coils to simplify the process and to save time and cost.

### SUMMARY

The present disclosure provides an improved occluding device for occlusion of fluid flow through a lumen of a body vessel. The occluding device includes a main coil having first primary coil windings with a first coil diameter and an end coil having second primary coil windings with a second coil diameter. The main coil is formed into a secondary coil with various shapes. The main coil has an initial tension of between about 5 and 60 grams of weight.

The second coil diameter of the end coil is greater than the first coil diameter of the main coil so the end coil can fit more tightly within a catheter tubing to prevent a wire guide being stuck between the occluding device and the catheter tubing.

The main coil and the end coil are monolithically formed from a single wire so the process of production is simplified and the risk of detachment of the end coil or main coil from the occluding device is avoided.

In one aspect, the main coil has a uniform first coil diameter and the end coil has a uniform second coil diameter. In other aspects, the main coil and the end coil each can have varied coil diameters.

The end coil can have a tapered shape such that the end coil increases its diameter gradually from the first coil diameter to the second coil diameter. The end coil can also taper twice such that the end coil increases its diameter gradually from the first coil diameter to the second coil diameter and then decreases gradually from the second coil diameter. The tapered shape of the end coil allows the primary windings with smaller diameter to fill the space in the end coil lumen so the wire guide does not get stuck easily between the coil windings and the catheter tubing. The wire guide can be advance more easily.

The end coil can also have one or a plurality of uniform portions with smaller coil diameter(s) than the second diameter to allow the one or more plurality of uniform portions to fill the lumen of the end coil portion with the second diameter.

The end coil can also have sections with offsetting central axes. One central axis of the end coil can be coincident with a central axis of the main coil and another central axis of the end coil can be parallel to the central axis of the main coil.

The present disclosure also includes an improved method of manufacturing the occluding device. The method includes winding a metal wire having a first portion and a second portion to form a main coil in the first portion, winding the main coil to a secondary coil, and winding the second portion of the metal wire to form an end coil. The main coil has first primary coil windings forming a first primary coil body with a first coil diameter, and the main coil has an initial tension of between about 5 and 60 grams of weight. The end coil has second primary coil windings forming a second primary coil body with a second coil diameter greater than the first coil diameter. The method provides a continuous process to make the occluding device with a main coil and an end coil from a single wire.

The present disclosure also provides a method of manufacturing at least two occluding devices for occlusion of fluid flow through a lumen of a body vessel in a continuous process. The method includes winding a metal wire having at least a first portion, a second portion and a third portion to form a first coil in the first portion having first primary coil windings with a first coil diameter, winding the second portion of the metal wire to form a second coil having second primary coil windings with a second coil diameter different from the first coil diameter, winding the third portion of the metal wire to form a third coil having third primary coil windings with a third coil diameter, and cutting the second coil to form a first occluding coil and a second occluding coil. The first occluding coil includes the first portion and one part of the second portion, and the second occluding coil has the third portion and the other part of the second portion. At least one of the first, second and third coils has an initial tension of between about 5 and 60 grams of weight.

To make multiple occluding devices, the method further includes continuing winding the metal wire to form multiple coils, and cutting the multiple coils to provide multiple embolization coils. Each of the embolization coils includes a portion of at least two of the multiple coils, and the at least two of the multiple coils have different coil diameters.

Other objects, systems, methods, features and advantages of the invention will become apparent to one of ordinary skill in the art from consideration of the following description and the appended claims when taken in connection with the accompanying drawings.

### BRIEF DESCRIPTION OF THE FIGURES

The invention can be better understood with reference to the following figures and description. The components in the figures are not necessarily drawn to scale, emphasis instead being placed upon illustrating the principles of the invention. Moreover, in the figures, like referenced numerals designate corresponding parts throughout the different views.
Fig. 1 is a side view of an occluding device according to one embodiment of the present disclosure.
Fig. 2 is a side view of an occluding device according to another embodiment of the present disclosure.
Fig. 3 is a side view of an occluding device according to yet another embodiment of the present disclosure.
Fig. 4 is a side view of an occluding device according to yet another embodiment of the present disclosure.
Fig. 5 is a side view illustrating an example of an embodiment of a method for producing the occluding device.
Fig. 6 is a side view of an occluding device according to yet another embodiment of the present disclosure.
Fig. 7 is a side view of an occluding device according to yet another embodiment of the present disclosure.
Fig. 8 is a side view of an occluding device according to yet another embodiment of the present disclosure.
Fig. 9 is a side view of an occluding device according to yet another embodiment of the present disclosure.
Fig. 10 is a side view of an occluding device according to yet another embodiment of the present disclosure.
Fig. 11 is a side view of an occluding device according to yet another embodiment of the present disclosure.
Fig. 12 is a side view of an occluding device according to yet another embodiment of the present disclosure.
Fig. 13 is a side view of an occluding device according to yet another embodiment of the present disclosure.
Fig. 14 is an exploded view of an embolization kit for one embodiment of the occluding device of the present disclosure.

### DETAILED DESCRIPTION

The present disclosure provides a medical embolization coil with an end coil and a main coil and a method of making such a coil. The materials, methods, figures, and examples disclosed herein are illustrative only and not intended to be limiting.

In the present application, the term "proximal" refers to a direction that is generally towards a physician during a medical procedure, while the term "distal" refers to a direction that is generally towards a target site within a patient's anatomy during a medical procedure. The terms "substantially" or "about" as used herein include variations in the recited characteristic or quantity that are functionally equivalent to the quantity recited, such as an amount that is equivalent to the quantity recited for an intended purpose or function. In the case of a numerical quantity, the terms "substantially" or "about" shall mean a range consisting of a value 20% less than the recited value to a value 20% greater than the recited value, inclusive.

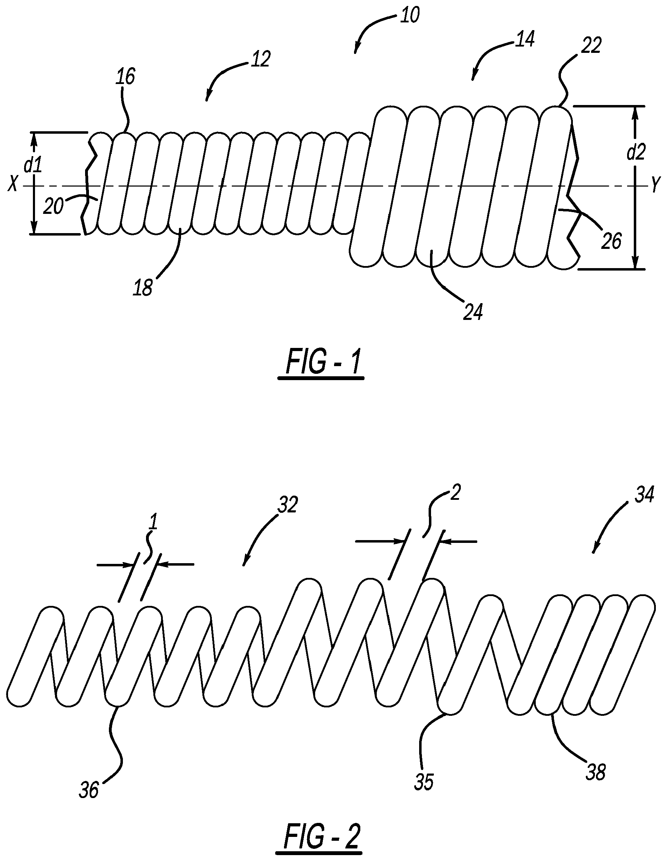

With reference to the Fig. 1, one embodiment of an occluding device 10 in accordance with the present invention is provided. The occluding device includes a main coil 12 and an end coil 14. The main coil 12 is formed by primary windings 16 that are arranged adjacent to each other. The primary windings 16 form a primary body 18 and surround a primary lumen 20. The end coil 14 is formed by primary windings 22 that are arranged adjacent to each other. The primary windings 22 form a primary body 24 and surround a primary lumen 26. Fig. 1 also shows coil 12 has a central axis x that is coincident with the central axis y of the end coil 14.

The two coils 12, 14 have different coil diameters. Fig. 1 shows coil 14 has a coil diameter d2 that is larger than the coil diameter d1 of the main coil 12, and each coil has a uniform diameter. The larger diameter of the end coil 14 provides greater rigidity or radial force, and also makes the end coil 12 fit more tightly within the a catheter tubing such that a wire guide does not get stuck easily between the occluding device 10 and the catheter tubing when the device is delivered through a catheter. The smaller main coil diameter allows for fibers to fit between the main coil 12 and the catheter for easier delivery. The smaller diameter also allows for tighter coil windings or curls. A typical length of the end coil 14 is about 2-5 millimeters. The main coil has a much larger length, for example, 3-20 centimeters, so it can be used for sufficient occlusion in a body vessel.

Fig. 1 shows both the main coil 12 and the end coil 14 are tightly wound such that adjacent primary windings 16, 22 are in close contact with each other. Such tight windings allow a friction fit between the windings for fibers to be held between the windings and not to slip out of the coil easily. It is also envisaged that the primary coil windings 16, 22 can have pitch intervals. As shown in Fig. 2, main coil 32 forms a predetermined interval 1 between the windings 36. End coil 34 has one section with a predetermined interval 2 between windings 35 and one section with tightly wound windings 38 such that the adjacent windings touch each other. Other configurations with varying pitch intervals between the windings are also possible.

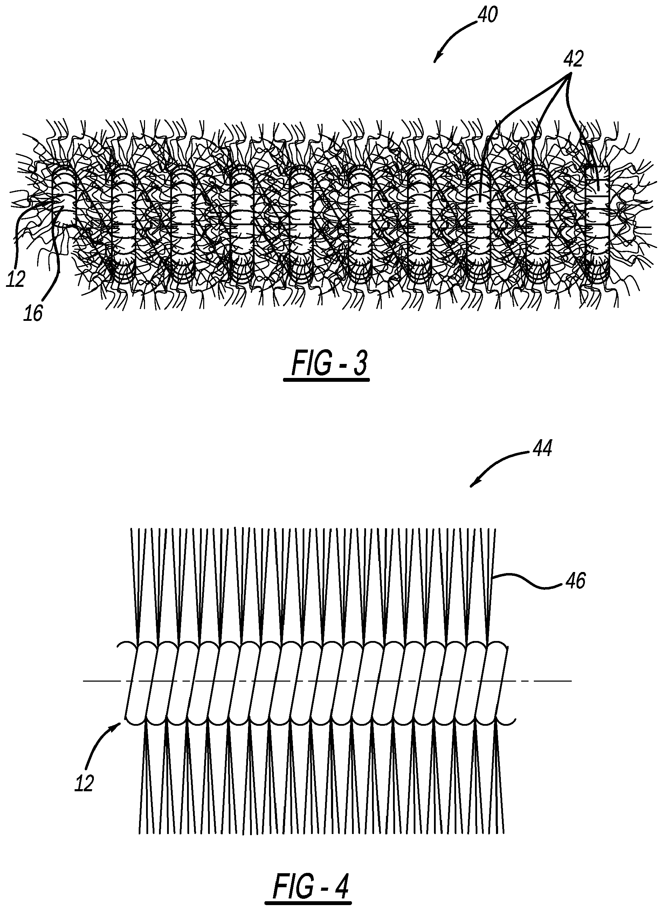

The embolization coil 10 can be further curled to form a secondary shape. Fig. 3 illustrates main coil 12 is shaped into a secondary helical coil 40. The secondary helical coil 40 includes a series of connected secondary loops 42 axially spaced apart by a predetermined distance. The series of secondary loops 42 can fill a cross-sectional area of the body vessel to occlude fluid flow through the lumen of the body vessel. Various secondary shapes may be form by the main coil 12 such as tornado or nest shapes, or other irregular secondary shapes with different diameters at different sections. The end coil may be curled together with the main coil to form the secondary shape; or only the main coil is curled. Because the end coil is 2-5 mm in length, the end coil may keep the shape being substantially straight.

Main coil 12 may have a pre-curled tension to facilitate main coil 12 being curled within the body vessel. The pre-curled tension, sometimes referred to as initial tension, may be defined to be the amount of force required to cause a 4 centimeter length of coil to begin to elongate. In one example, the main coil 12 has a pre-curled tension of between about 5 to 60 grams of weight, and preferably between about 10 to 30 grams of weight. In another example, the main coil 12 has a pre-curled tension of between about 65 to 120 grams of weight, and preferably between about 75 to 100 grams of weight. A coil having a pre-curled tension may be restrained substantially straight (see Fig. 1), e.g., for advancing through a catheter arrangement for deployment, and reforming or recanalizing back to a coiled, looping or curled configuration when the restraining force is released, e.g., after deployed from the catheter into the body vessel.

The occluding device may include attached fibers extending therefrom. The fibers may be spaced apart from each other and held between primary windings 16 of the main coil 12, as shown in Figs. 3 and 4. The fibers 44 may include strands 46 comprising a synthetic polymer such as polyester textile fiber, e.g., DACRON™. As desired, the strands 46 may be positioned between adjacent loops, alternating loops, alternating double loops, or any desired configuration. Preferably, the strands 46 have a length extending from the main coil 12 between 5 to 6 millimeters as desired. In this embodiment, the fibers 44 are spaced apart from each other by about 1 to 2 millimeters. Preferably, the strands 46 have an outer diameter of about 0.00050 to 0.00100 inch.

As mentioned previously, the current process of making the embolization coil 10 by making separate main coil and end coil followed by an attachment step requires more time and cost and may have the risk of detachment. This disclosure provides a single process of making the embolization coil 10 by a continuous process from a single wire so the main coil and the end coil are monolithically formed. The process saves time and eliminates the risk of a loose part.

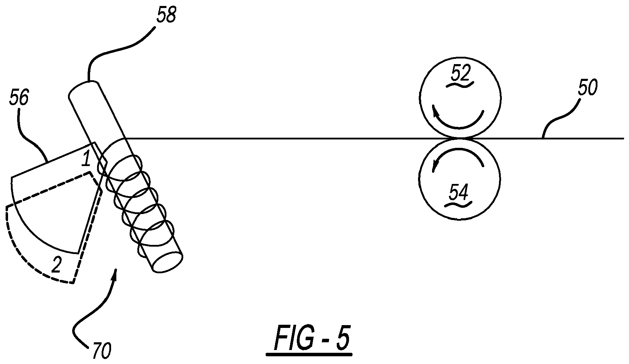

In this process, the end coil is incorporated into the embolization coil during the coiling process. Fig. 5 illustrates the continuous process for making the embolization coil with a main coil and an end coil. A single wire 50 is fed between an upper roller 52 and a lower roller 54. The rollers rotate in the directions of the arrows in Fig. 5 to drive the wire 50 through to reach a coiling area 70. The coiling area 70 has a coiling point 56 and a rounded tool 58. Wire 50 reaches the coiling area 70 and makes contact with the rounded tool 58, and then is deflected by the coiling point 56 to form an arc or circle. The formed arc or circle wraps around the rounded tool 58. With wire 50 being fed through the rollers continuously to the coiling area, the wire 50 is deflected by the coiling point 56 continuously to form a coil with a uniform coil diameter. The diameter of the coil is determined by the location of the coiling point 56 or the point of contact of the wire with the coiling point 56. By adjusting the coiling point 56 towards the rollers or away from the rollers, or by adjusting the coiling point 56 up or down, the diameter of the coil can be adjusted. Therefore, embolization coil 10 can be made by feeding the wire 50 through the rollers while maintaining the coiling point 56 at position 1. Wire 50 is coiled to make the main coil 12 first with uniform coil diameter d1 until the desired length for the main coil is reached. The process continues and the coiling point 56 is adjusted to position 2, away from the rollers, so the wire 50 is deflected to a greater diameter coil to form the end coil 14 with diameter d2. In this way, the embolization coil 10 is made with a continuous process without a separate attachment step. Main coil 12 can be made first followed by end coil 14, or end coil 14 can be made first with the coiling point at position 2 first followed by main coil 12. Wire 50 can be tightly wound so the windings touch each other, or the wire can be wound to produce intervals between the windings.

The material for the metal wire 50 is not particularly limited. Examples of the material may include stainless steel, platinum, tungsten, gold, tantalum, iridium, titanium, palladium, nickel, platinum alloys, cobalt-chromium-nickel-molybdenum-iron alloy, cobalt-chrome alloy, and Inconel alloy (Ni-Cr alloy). The cross-sectional shape of wire 50 is not limited to a circular shape. Various shapes like square shape, elliptical shape and other shapes may be selected.

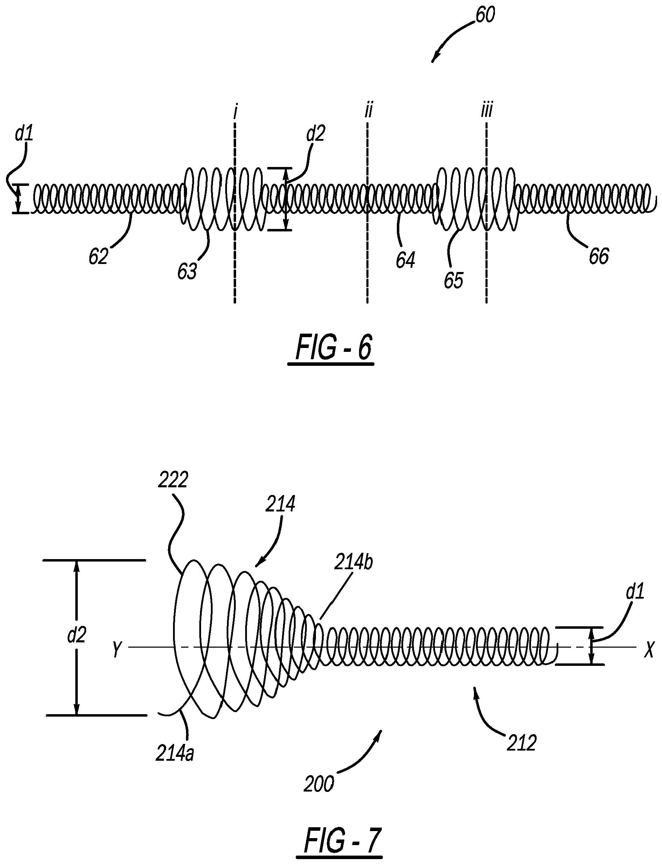

Fig. 6. shows a coil 60 with multiple sections of main coils and end coils made in a continuous process. Each main coil section and each end coil section has primary windings similar to that shown in Fig. 1. Although Fig. 6 shows all the main coil sections 62, 64, 66 have uniform coil diameter d1, and all the end coil sections 63, 65 have uniform coil diameter d2, all the coil sections can have different diameters for any particular purpose, and may not be uniform. The length of each section of coil 60 may be varied according to particular uses for coil 60.

Coil 60 can be made from a single wire 90 by a process similar to the process illustrated in the Fig. 5, except during the process, the position of the coiling point is adjusted several times to make different sections of the coil for different diameters while wire 90 is fed through the rollers continuously. Wire 90 is similar to wire 50 except wire 90 may have a greater length than wire 50, or a different wire diameter.

Coil 60 can be used as an occluding device as it is. Multiple occluding devices 10 can also be made from coil 60. For this purpose, the length of each end coil 63, 65 is about 4-10 mm. The main coils 62 and 66 have a length that is similar to the length of main coil 12, and section 64 has a length that is about twice the length of main coil 12. Mandrel 70 is thus designed to have sections corresponding to the length requirement. After coil 60 is made, coil 60 is cut at line i at end coil section 63, line ii at main coil section 64, and line iii at end coil section 65 to afford four embolization coils. Each embolization coil has a main coil and an end coil, similar to embolization coil 10, with the proper lengths for the main coil and the end coil. Therefore, this continuous process of making embolization coil 10 with an end coil and a main coil saves multiple attachment steps and time to produce multiple embolization coils.

Longer coils with more main coil sections and end coil sections than coil 60 can be made with the continuous process. More embolization coils 10 can be made as illustrated above by cutting the long coil at multiple locations. Thus, more time, effort and cost can be saved.

Figs. 7-12 show different embodiments of the embolization coil with an end coil and a main coil. In all the figures shown, the main coil has a uniform diameter d1, and the end coil has a section with a coil diameter that is greater than the main coil diameter d1. The figures are for illustration purpose only and for simplicity. The main coils can have varying diameters along its length, and the figures are not intended to limit the configurations of the main coil to a uniform diameter. The figures show tightly wound primary windings, but varying pitch intervals are also within the scope of the disclosure.

Fig. 7 shows an embolization coil 200 with a tapered end coil 214. End coil 214 has its largest coil diameter d2 at one end 214a, and then end coil 214 tapers down along its central axis y. The main coil 212 has a uniform diameter d1 and central axis x coincident with axis y. The primary windings 222 of end coil 214 have gradually decreased diameters so end coil 214 has a conically helical shape and the end coil tapers down from its largest diameter d2 at the end 214a to a diameter equal to the main coil's diameter d1 at another end 214b.

Embolization coil 200 can be made from a single wire by a single process. During the process, in one approach, the coiling point is at a position furthest from the roller so the wire is deflected to have a coil diameter d2 first. The coiling point is then moved at a constant speed closer to the rollers while the wire is in contact with the coiling point. The wire is thus coiled to have tapered coil diameters to form the tapered end coil 214. Once the coiling point is at a position where the resulting coil diameter is d1, the coiling point stops moving and the wire is then deflected to the main coil 212 to have a constant diameter d1. In another approach, the coiling point can be at the position closest to the roller to make the main coil 212 first with diameter d1, and then is moved away from the rollers to produce the tapered end coil 214.

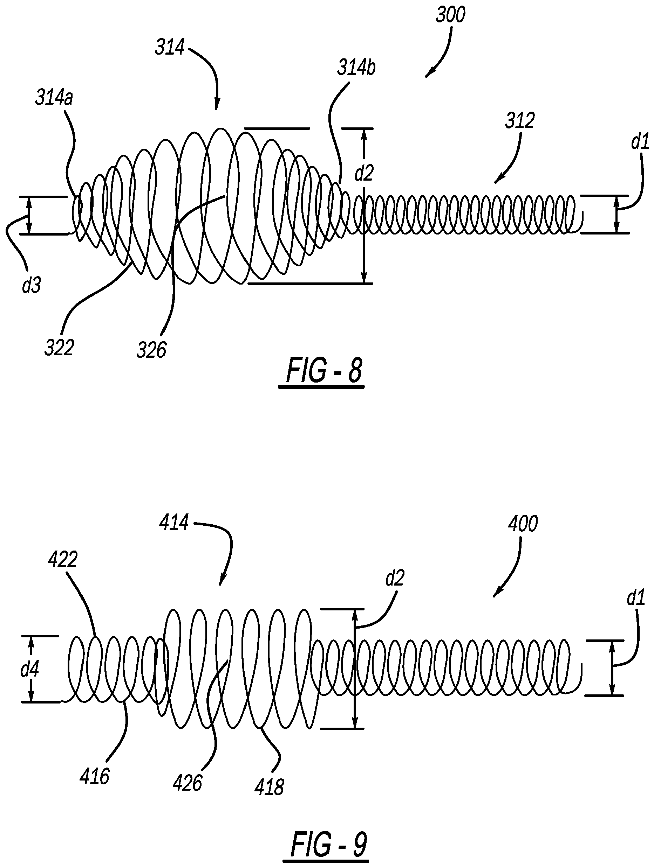

Fig. 8 shows an embolization coil 300 with an end coil that tapers twice. End coil 314 has an initial diameter d3 at one end 314a. The primary windings 322 of end coil 314 have gradually increased diameters from d3 to d2. The end coil 314 then tapers down and the diameters decrease from d2 to d1, which is the diameter of main coil 312. Diameters d1 and d3 may be the same or different. In this embodiment, the central axes of end coil 314 and the main coil 312 are coincident with each other. End coil 314 has a primary lumen 326 defined by primary windings 322. One benefit provided by the tapered shape is that primary windings with smaller diameters can fill the space in primary lumen 326 when the occluding device 300 is delivered through a catheter. Therefore, empty space is filled with the primary winds and the wire guide does not get stuck between the embolization coil and the catheter when the wire guide is advanced through.

Embolization coil 300 can be made from a single wire by a similar process illustrated previously.

Fig. 9 shows an embolization coil 400 with an end coil 414 having two sections of different diameters. Section 416 has a constant diameter of d4, and section 418 has a constant diameter of d2. Diameter d2 is greater than d4, and d4 may be the same as or different from the main coil diameter d1. Similar to the embolization coil 300, the primary windings 422 of section 416 can fill the primary lumen 426 of the coil in section 418 so that the wire guide can be advanced more easily. The central axes of section 416, 418 and main coil 412 are coincident with each other.

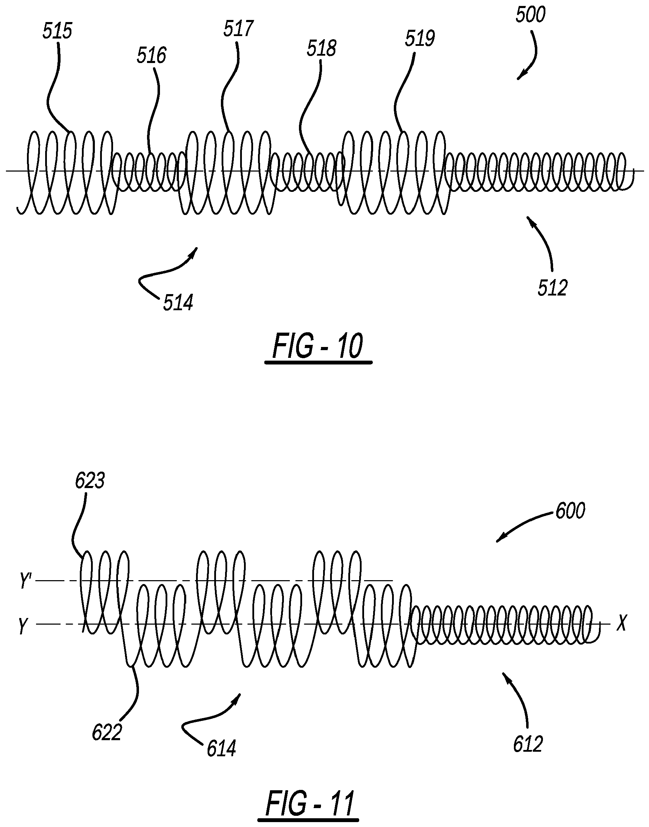

Fig. 10 shows and embolization coil 500 with an end coil 514 having multiple sections of different diameters. In this embodiments, all the sections including main coil 512 have coincident central axes. The larger diameter sections 515, 517, and 519 of the end coil 512 may have the same diameter, for example, d2, or they have different diameters from each other. Sections 515, 517, and 519 all have larger diameters than the smaller sections 516 and 518. Section 516 and 518 may have the same or different diameters. They may also have the same diameter as the main coil 512. Primary windings in section 516 and 517 can fill the lumen of sections 515, 518 and 519 to prevent the wire guide getting stuck in the coil.

Fig. 11 shows an embolization coil 600 with an end coil 614 having sections with offsetting central axes. In this embodiment, end coil 614 has two sets of primary windings 622 and 623. Winding set 622 has a central axis y that is coincident with the central axis x of main coil 612. Winding set 623 has a central axis y' that is parallel to central axis y. windings 622 and 623 may have the same diameter or different diameters. The offsetting central axes of two winding sets allow windings 622 to fill the lumen of winding 623 and windings 623 to fill the lumen of winding 622. It is also possible the central axis x of main coil is not coincident, but parallel to central axis y.

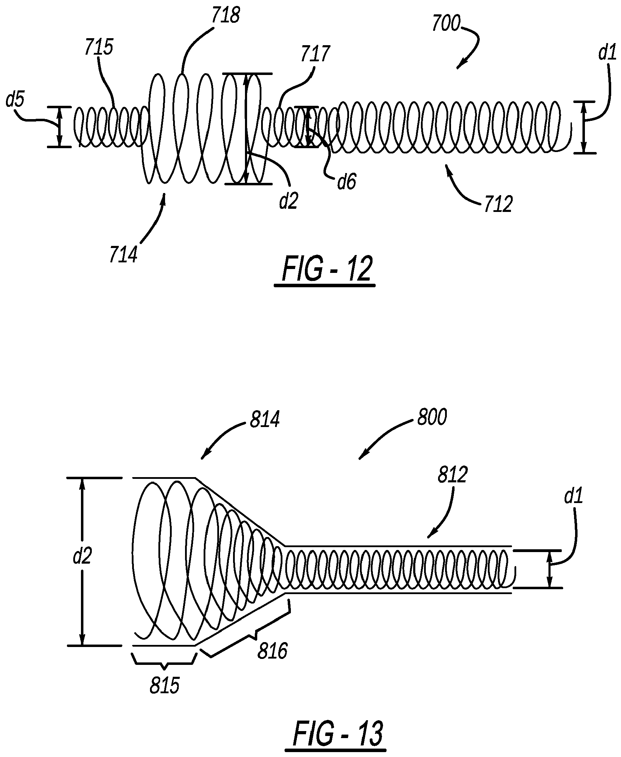

Fig. 12 shows another embodiment of an embolization coil 700 with an end coil 714 having sections of different diameters. End coil 714 has two smaller diameter sections 715 and 717, and one larger diameter section 718. The diameters d5, d6 of section 715 and 717 may be the same or different, and they may be smaller than the main coil diameter d1. The larger diameter section 718 has a uniform diameter d2 that is greater than d1, d5 and d6. The lumen of section 718 is closed off by section 715 and 717 coils to facilitate the advancement of the wire guide. The central axes of different sections are coincident in this embodiment.

Fig. 13 shows another embodiment of an embolization coil 800 with an end coil 814 having a tapered section 816 and a straight or uniform section 815. The uniform section 815 has a diameter d2, larger than the diameter of the rest of the end coil 814 and the diameter of the main coil 812. From the uniform section 815, the end coil tapers down, and the coil windings have decreased diameters from d2 to the main coil diameter d1. In this embodiment, the main coil 812 has uniform diameter d1 and the central axes of different sections are coincident. It is also envisaged that the main coil 812 may have tapered section too and different coil sections may have parallel axes.

In this embodiment, the straight or uniform section 815, with its larger diameter, provides greater radial force or rigidity to allow the end coil to fit more tightly within the catheter tubing than embolization coils with only tapered end coil. Also, the straight portion helps positioning and orienting the whole embolization coil within the catheter, so the embolization coil is not tilted to either side. The tapered section 816 then can fill the lumen of section 815 to facilitate the wire guide advancing along the central axis of the embolization coil without being wedged between the coil and the catheter tubing. The combination of the straight portion and the tapered portion for the end coil thus benefits the deployment of the embolization coil.

Different embodiments of the embolization coil can be made from a single wire by a similar process illustrated previously. By adjusting the position or the point of contact of the wire with the coiling point, the resulting coil can have varying coil diameters suitable for particular purposes.

Multiple embolization coils of the above mentioned embodiments can be made with a continuous process. An embolization coil with multiple main coils and multiple end coil sections can be made with this process, and then the embolization coil can be cut at multiple sections to afford multiple coils having an end coil and a main coil.

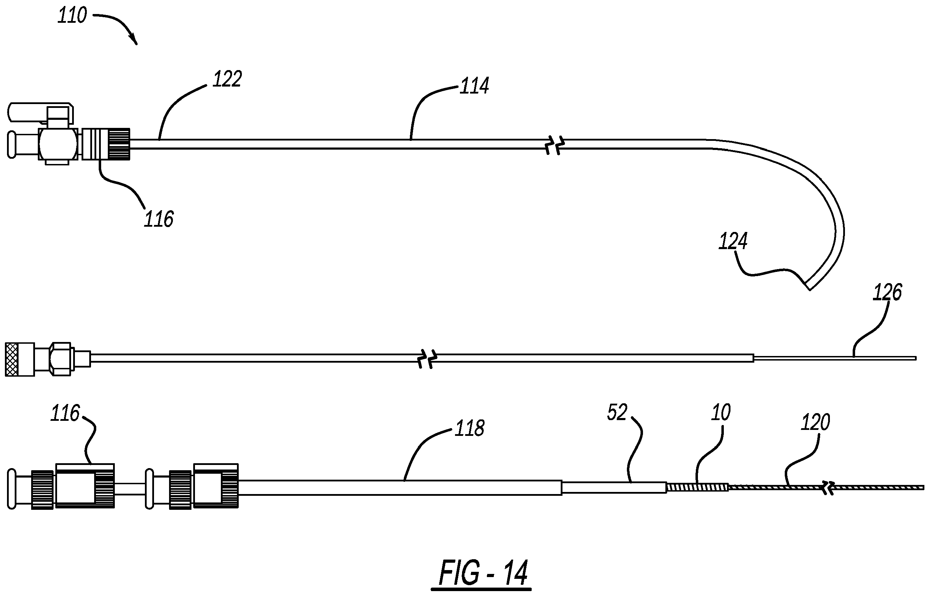

Fig. 14 illustrate an embolization kit 110 which implements the occluding device in accordance with one embodiment of the present invention. As shown, the kit 110 includes a microcatheter 114 preferably made from a soft, flexible material such as silicone or any other suitable material. Generally, the microcatheter 114 has a proximal end 122, a distal end 124, and a plastic adapter or hub 116 to receive the device 10 to be advanced therethrough. In this embodiment, the inside diameter of the microcatheter 114 may range between 0.014 and 0.027 inch. The kit 110 further includes a wire guide 120 which provides a guide catheter 118 a path during insertion of the guide catheter 118 within the body vessel. The size of the wire guide 120 is based on the inside diameter of the guide catheter 118.

The guide catheter 118 or sheath is made of polytetrafluoroethylene (PTFE) for percutaneously introducing the microcatheter 114 into the body vessel. Of course, any other suitable material may be used without falling beyond the scope or spirit of the present invention. The guide catheter 118 may have a size of between about 4-French to 8-French and allows the microcatheter 114 to be inserted therethrough to a desired location in the body vessel. The guide catheter 118 receives the microcatheter 114 and provides stability of the microcatheter 114 at a desired location within the body vessel. For example, the guide catheter 118 may stay stationary within a common visceral artery, e.g., a common hepatic artery, and adds stability to the microcatheter 114 as the microcatheter 114 is advanced through the guide catheter 118 to a point of occlusion in a connecting artery, e.g., the left or right hepatic artery.

When the distal end 124 of the microcatheter 114 is at the point of occlusion in the body vessel, the occluding device 10 is loaded at the proximal end 122 of the microcatheter 114 and is advanced through the microcatheter 114 for deployment through the distal end 124. In this embodiment, a push wire 126 is used to mechanically advance or push the occluding device 10 through the microcatheter 114. The size of the push wire 126 used depends on the diameter of the microcatheter 114.

It is to be understood that the body cavity embolization kit 110 described above is merely one example of a kit that may be used to deploy the occluding device 10 in a body vessel. Of course, other kits, assemblies, and systems may be used to deploy any embodiment of the occluding device without falling beyond the scope or spirit of the present invention.

The initial tension described herein may represent the amount of force necessary to cause a 4 centimeter length of the main coil to elongate, for example to begin to elongate. The main coil may be helical, and elongating may comprise elongation of the helix. Initial tension may comprise the difference between (a) an amount of force (e.g. tensile load) on the spring, and (b) the product of its spring constant and the elongation due to the amount of force (e.g. tensile load).

In an embodiment there is provide an occluding device for occlusion of fluid flow through a lumen of a body vessel, the occluding device comprising: a main coil having first primary coil windings forming a first primary coil body with a first coil diameter, the main coil having an initial tension of between about 5 and 60 grams of weight, the main coil being formed into a secondary coil; and an end coil having second primary coil windings forming a second primary coil body with a second coil diameter greater than the first coil diameter, the main coil and the end coil being monolithically formed. All the first primary coil windings may have the first coil diameter and all the second primary coil windings may have the second coil diameter. The end coil may have a tapered shape such that the second primary coil windings increase diameters gradually from the first coil diameter to the second coil diameter. The end coil may taper twice such that the second primary coil windings increase diameters gradually from the first coil diameter to the second coil diameter and then decrease gradually from the second coil diameter. The end coil may have a first uniform portion having the second coil diameter and a second uniform portion having a fourth coil diameter, the second diameter being greater than the fourth diameter. The end coil may have a first plurality of uniform portions having the second coil diameter and a second plurality of uniform portions having a fourth coil diameter, each of the second plurality of uniform portions being disposed between each of the first plurality of uniform portions. The end coil may have one central axis that is coincident with a central axis of the main coil and another central axis that is parallel to the central axis of the main coil. The end coil ma have a tapered section and a uniform portion, and the second primary coil windings increase diameters gradually from the first coil diameter to the second coil diameter in the tapered section and remain the second coil diameter constant in the uniform portion.

An embodiment provides a method of manufacturing an occluding device for occlusion of fluid flow through a lumen of a body vessel, the method comprising: winding a metal wire having a first portion and a second portion to form a main coil in the first portion, the main coil having first primary coil windings forming a first primary coil body with a first coil diameter, the main coil having an initial tension of between about 5 and 60 grams of weight; winding the main coil to a secondary coil; and winding the second portion of the metal wire to form an end coil having second primary coil windings forming a second primary coil body with a second coil diameter greater than the first coil diameter, the main coil and the end coil being monolithically formed.

The initial tension may comprise an amount of force necessary to cause a 4 centimeter length of the main coil to begin to elongate. The first primary coil windings may have the first coil diameter and all the second primary coil windings may have the second coil diameter. The end coil may have a tapered shape such that the second primary coil windings increase diameters gradually from the first coil diameter to the second coil diameter. The end coil may taper twice such that the end coil increases its diameter gradually from the first coil diameter to the second coil diameter and then decreases gradually from the second coil diameter. The end coil may have a first uniform portion having the second coil diameter and a second uniform portion having a fourth coil diameter, the second diameter being greater than the fourth diameter. The end coil may have a tapered section and a uniform portion, and the second primary coil windings increase diameters gradually from the first coil diameter to the second coil diameter in the tapered section and remain the second coil diameter constant in the uniform portion. The metal wire may further comprise a third portion, and winding the main wire may comprise forming a first coil in the first portion (the main coil mentioned above may be provided by part of this first coil by the cutting mentioned below). The method may comprise winding the second portion of the metal wire to form a second coil having second primary coil windings forming a second primary coil body with a second coil diameter different from the first coil diameter. The method may comprise winding the third portion of the metal wire to form a third coil having third primary coil windings forming a third primary coil body with a third coil diameter. The method may comprise cutting the second coil to form a first occluding coil and a second occluding coil, the first occluding coil having the first portion and a part of the second portion, the second occluding coil having the third portion and another part of the second portion, at least one of the first, second and third coils having an initial tension of between about 5 and 60 grams of weight.

An embodiment provides a method of manufacturing at least two occluding devices for occlusion of fluid flow through a lumen of a body vessel in a continuous process, the method comprising: winding a metal wire having at least a first portion, a second portion and a third portion to form a first coil in the first portion, the first coil having first primary coil windings forming a first primary coil body with a first coil diameter; winding the second portion of the metal wire to form a second coil having second primary coil windings forming a second primary coil body with a second coil diameter different from the first coil diameter; winding the third portion of the metal wire to form a third coil having third primary coil windings forming a third primary coil body with a third coil diameter; cutting the second coil to form a first occluding coil and a second occluding coil, the first occluding coil having the first portion and a part of the second portion, the second occluding coil having the third portion and another part of the second portion, at least one of the first, second and third coils having an initial tension of between about 5 and 60 grams of weight; and winding the at least one of the first, second and third coils to a secondary coil. The initial tension may be an amount of force necessary to cause a 4 centimeter length of the at least one of the first, second and third coils to begin to elongate. The first coil diameter and the third coil diameter are the same, and the second coil diameter is larger than the first coil diameter.

The method may further comprise continuing winding the metal wire to form multiple coils, each of the multiple coils having a coil diameter different from an adjacent coil's coil diameter; cutting the multiple coils to provide multiple embolization coils, each of the embolization coils comprising a portion of at least two of the multiple coils, the at least two of the multiple coils having different coil diameters.

The second coil has a tapered shape such that the second coil increases its diameter gradually from the first coil diameter to the second coil diameter. The second coil may have a first uniform portion having the second coil diameter and a second uniform portion having a fourth coil diameter, the second diameter being greater than the fourth diameter. The second coil may have one central axis that is coincident with a central axis of the first coil and another central axis that is parallel to the central axis of the first coil.

As a person skilled in the art will readily appreciate, the above description is meant as an illustration of the implementation of the principles of this invention. This description is not intended to limit the scope of application of this invention in that the invention is susceptible to modification, variation, and change, without departing from the spirit of this invention, as defined in the following claims.

## Claims

1. An occluding device for occlusion of fluid flow through a lumen of a body vessel, the occluding device comprising:
a main coil having first primary coil windings forming a first primary coil body with a first coil diameter, the main coil having an initial tension of between about 5 and 60 grams of weight, the main coil being formed into a secondary coil; and
an end coil having second primary coil windings forming a second primary coil body with a second coil diameter greater than the first coil diameter, the main coil and the end coil being monolithically formed.

2. The occluding device of claim 1, wherein the end coil has one central axis that is coincident with a central axis of the main coil and another central axis that is parallel to the central axis of the main coil.

3. The occluding device of claim 1 or 2, wherein the end coil has a tapered section and a uniform portion, and the second primary coil windings increase diameters gradually from the first coil diameter to the second coil diameter in the tapered section and remain the second coil diameter constant in the uniform portion.

4. A method of manufacturing an occluding device for occlusion of fluid flow through a lumen of a body vessel, the method comprising:
winding a metal wire having a first portion and a second portion to form a main coil in the first portion, the main coil having first primary coil windings forming a first primary coil body with a first coil diameter, the main coil having an initial tension of between about 5 and 60 grams of weight;
winding the main coil to a secondary coil; and
winding the second portion of the metal wire to form an end coil having second primary coil windings forming a second primary coil body with a second coil diameter greater than the first coil diameter, the main coil and the end coil being monolithically formed.

5. The method of claim 4, or the occluding device of any of claims 1 to 3, wherein all the first primary coil windings have the first coil diameter and all the second primary coil windings have the second coil diameter.

6. The method of claim 4 or 5, or the occluding device of any of claims 1 to 3 wherein the end coil has a tapered shape such that the second primary coil windings increase diameters gradually from the first coil diameter to the second coil diameter.

7. The method of claim 4, 5, or 6 or the occluding device of any of claims 1 to 3, wherein the end coil tapers twice such that the end coil increases its diameter gradually from the first coil diameter to the second coil diameter and then decreases gradually from the second coil diameter.

8. The method of any of claims 4 to 7, or the occluding device of any of claims 1 to 3, wherein the end coil has a first uniform portion having the second coil diameter and a second uniform portion having a fourth coil diameter, the second diameter being greater than the fourth diameter, for example wherein the end coil has a first plurality of uniform portions having the second coil diameter and a second plurality of uniform portions having a fourth coil diameter, each of the second plurality of uniform portions being disposed between each of the first plurality of uniform portions..

9. The method of any of claims 4 to 8, wherein the end coil has a tapered section and a uniform portion, and the second primary coil windings increase diameters gradually from the first coil diameter to the second coil diameter in the tapered section and remain the second coil diameter constant in the uniform portion.

10. A method of manufacturing at least two occluding devices for occlusion of fluid flow through a lumen of a body vessel in a continuous process, the method comprising:
winding a metal wire having at least a first portion, a second portion and a third portion to form a first coil in the first portion, the first coil having first primary coil windings forming a first primary coil body with a first coil diameter;
winding the second portion of the metal wire to form a second coil having second primary coil windings forming a second primary coil body with a second coil diameter different from the first coil diameter;
winding the third portion of the metal wire to form a third coil having third primary coil windings forming a third primary coil body with a third coil diameter;
cutting the second coil to form a first occluding coil and a second occluding coil, the first occluding coil having the first portion and a part of the second portion, the second occluding coil having the third portion and another part of the second portion, at least one of the first, second and third coils having an initial tension of between about 5 and 60 grams of weight; and
winding the at least one of the first, second and third coils to a secondary coil.

11. The method of claim 10, wherein the first coil diameter and the third coil diameter are the same, and the second coil diameter is larger than the first coil diameter.

12. The method of claim 10 or 11, further comprising:
continuing winding the metal wire to form multiple coils, each of the multiple coils having a coil diameter different from an adjacent coil's coil diameter;
cutting the multiple coils to provide multiple embolization coils, each of the embolization coils comprising a portion of at least two of the multiple coils, the at least two of the multiple coils having different coil diameters.

13. The method of claim 10, 11, or 12, wherein the second coil has a tapered shape such that the second coil increases its diameter gradually from the first coil diameter to the second coil diameter.

14. The method of any of claims 10 to 13, wherein the second coil has a first uniform portion having the second coil diameter and a second uniform portion having a fourth coil diameter, the second diameter being greater than the fourth diameter.

15. The method of any of claims 10 to 14, wherein the second coil has one central axis that is coincident with a central axis of the first coil and another central axis that is parallel to the central axis of the first coil.
